# EUROPEAN PATENT SPECIFICATION

(11) **EP 4 523 666 B1**
(45) Date of publication and mention of the grant of the patent: **28.01.2026**
(21) Application number: 23197264.7
(22) Date of filing: 13.09.2023
(51) Int. Cl.: A61F 13/02, A61F 13/05, A61F 13/0203, A61M 1/00

(54) **A WOUND DRESSING**
WUNDVERBAND
PANSEMENT POUR PLAIES

(43) Date of publication of application: 19.03.2025
(73) Proprietor: Mölnlycke Health Care AB, 431 21 Mölndal (SE)
(72) Inventor: SÖDERSTRÖM, Bengt, 435 40 MÖLNLYCKE (SE)
(74) Representative: Kransell & Wennborg KB

(56) References cited:
- WO-A1-2014/140606
- WO-A1-2021/033051
- US-B2- 10 772 999

## Description

### TECHNICAL FIELD

The present disclosure generally relates to a wound dressing comprising a backing layer, an adhesive skin contact layer and a wound pad arranged between the backing layer and the adhesive skin contact layer, wherein the backing layer and the adhesive skin contact layer are arranged to extend beyond the contour of the wound pad to form a border portion.

### BACKGROUND

The production of wound exudate is a natural part of the wound healing process. Excessive production of wound exudate may, however, be an indication of wound infection, and is often associated with chronic wounds.

Absorbent wound dressings are frequently used to treat various types of exuding wounds. In recent years, the design and construction of absorbent wound dressings have been improved for the purpose of enhancing their overall exudate handling capacity.

An absorbent wound dressing may comprise a plurality of layers and a variety of materials, optimized for handling large amounts of wound exudate.

The advancements in the design of wound dressings are generally associated with increased costs. In this regard, wear time is important. A wound dressing should preferably stay on the skin and/or wound of a patient during a prolonged period to avoid unnecessary dressing changes and costs.

One challenge with absorbent wound dressings is that it may be difficult for a caregiver (and a patient) to understand when the dressing has reached its full capacity and needs to be changed and replaced with a new one.

Many times, the wound dressing is changed and discarded too early; i.e. before the wound dressing has reached its full capacity.

In some hospitals and care facilities, caregivers and medical personnel apply various protocols with respect to when a wound dressing should be changed. Such protocols typically involve a recommendation to change the dressing when the wound exudate has reached one, two, or sometimes three, of the peripheral edges of the wound pad. Generally, this recommendation is based on the risk for wound exudate leaking back towards the wound site due to saturation of the dressing. Back-flow of wound exudate may lead to maceration at the wound site and at the skin surrounding the wound site, which may result in a prolonged wound healing process.

However, many times, such protocols and recommendations are incorrect. The mere wetting of one (or more than one) peripheral edge of the wound pad does not, in fact, yield a proper indication of dressing saturation or a correct indication that the dressing must be replaced. WO2014/140606A1 relates to the treatment of wounds using dressings in combination with negative pressure wound therapy, and methods for sizing a dressing for use in treating a wound.

In view of this, there is a need to provide a wound dressing having an improved liquid handling capacity, and which allows for an improved guidance to caregivers and patients as to when the dressing has reached its full capacity and needs to be changed.

### SUMMARY

In view of the above-mentioned problems, it is an object of the present disclosure to provide improvements in the field of absorbent wound dressings allowing for a facilitated guidance as to when the dressing needs to be replaced with a new one such that the overall wear time of the dressing is enhanced.

According to a first aspect of the present disclosure, there is provided a wound dressing, as defined in claim 1, comprising a backing layer, an adhesive skin contact layer and a wound pad arranged between the backing layer and the adhesive skin contact layer, wherein the backing layer and the adhesive skin contact layer are arranged to extend beyond the contour of the wound pad to form a border portion, wherein the wound pad comprises an upper portion and a lower portion; the upper portion being arranged in contact with the backing layer, wherein the upper portion has a surface area being smaller than the surface area of the lower portion and wherein the upper portion has a higher liquid spreading capacity than the lower portion.

The present disclosure is based on the realization that a wound dressing as defined hereinabove allows for an improved, greatly simplified and facilitated means to indicate to a caregiver when the wound dressing has reached its full capacity and needs to be replaced with a new dressing. Furthermore, the overall liquid handling capacity is improved, and the dressing can stay on the skin for a prolonged period.

Wound liquid exuded from the wound site is first acquired by the lower portion of the wound pad, which transports the exudate into the upper portion of the wound pad. The upper portion absorbs the liquid and has a higher liquid spreading capacity than the lower portion. Hence, liquid is efficiently spread and distributed across the surface area of the upper portion and will primarily be retained in the upper portion, until the dressing is saturated.

When the upper portion becomes saturated, the wound exudate will spread towards the underlying lower portion of the wound pad and become visibly observable on the edges of the lower portion of the wound pad (due to the smaller surface area of the upper portion). Hence, the mere wetting of one or several edges of the upper portion of the wound pad is not an indication of dressing saturation since there may still be significant capacity left in the wound pad. Instead, once the wound exudate reaches the lower portion of the wound pad, this is an indication of back-flow of exudate towards the wound site, which means that the dressing needs to be replaced.

Another advantage with a dressing as defined hereinabove is that wound exudate will be efficiently transported away from the dressing by evaporation. The higher liquid spreading capacity of the upper portion secures that exudate evaporates efficiently from the backing layer across a large surface area. Local saturation of wound exudate is thus avoided, and the overall liquid handling capacity and breathability of the dressing is improved.

The liquid spreading capacity of the upper portion is at least 50% higher, preferably at least 100% higher, more preferably at least 200% higher than the liquid spreading capacity of the lower portion.

The liquid spreading capacity gradient between the upper and the lower portion is preferably large. This is to improve the overall liquid handling capacity of the dressing and to secure that the wound exudate that is observable on the peripheral edges of the lower portion of the wound pad is indeed due to saturation of wound exudate. In other words, a proper indication that the dressing needs to be changed is provided.

The surface area of the upper portion corresponds to from 60 to 95%, preferably from 70 to 90% of the surface area of the lower portion.

Accordingly, the liquid handling capacity of the upper portion (and the entire dressing) is optimized, yet the back-flow of exudate (towards the lower portion) is still easily observable for a caregiver.

In exemplary embodiments, the upper portion is defined by peripheral edges and wherein the lower portion is arranged to extend around the peripheral edges of the upper portion.

Accordingly, the lower portion forms a "border portion" around the contour of the upper portion of the wound pad. The area formed between the peripheral edges of the lower portion and the peripheral edges of the upper portion forms a "wetness indicator area". The wound exudate may be visibly observable at each edge of the lower portion of the dressing. Accordingly, the dressing may be placed in any direction on the patient.

Typically, the upper portion is centrally disposed on the lower portion.

In exemplary embodiments, the lower portion is arranged to extend a distance, d1, around the peripheral edges of the upper portion, wherein the distance, d1, is from 3 to 20 mm, preferably from 4 to 12 mm.

An area in the above-mentioned range allows for wound exudate to be readily observable and serves as a reliable means to guide a caregiver that the dressing needs to be changed. Furthermore, the liquid capacity of the upper portion, and the entire dressing, is maintained.

In exemplary embodiments, the upper portion may have a higher liquid retention capacity than the lower portion.

Hence, the upper portion has an improved ability to retain the absorbed wound exudate. In this regard, liquid is further prevented from leaking back towards the wound site such that the skin and the wound site can be kept relatively dry. Maceration of the wound site is thereby avoided.

In exemplary embodiments, the upper portion may have a higher absorption capacity than the lower portion.

The overlying, more absorbent upper portion deals with the wound exudate and prevents the lower portion of the pad (and dressing) from becoming locally saturated. The overall liquid handling capacity, and the wear time of the dressing is thereby improved.

In exemplary embodiments, the lower portion may comprise a hydrophilic and porous material.

Hence, liquid is quickly and efficiently acquired by the lower portion, and the transport of the liquid towards the upper portion is enhanced. The liquid inlet time; i.e. the time it takes for liquid to be absorbed into the dressing, is improved.

In exemplary embodiments, the lower portion may comprise a polyurethane foam.

A polyurethane foam is a porous and hydrophilic foam with a rapid liquid acquisition rate. A polyurethane foam is capable of transporting the acquired liquid rapidly from the wound site towards the upper portion; i.e. in the vertical direction into the wound pad.

While a polyurethane foam has a good ability to transport exudate away from the wound site in directions substantially perpendicular to the plane thereof, a polyurethane foam typically has a relatively poor ability to spread and/or distribute the liquid in the transverse directions. A material which enables vertical liquid transport, but which has a poorer transverse liquid distribution capacity is generally preferred in the present disclosure.

Furthermore, a polyurethane foam is conformable and stretchable, which is beneficial as the lower portion is arranged in close proximity of the skin surface.

In exemplary embodiments, the upper portion comprises a superabsorbent material.

The superabsorbent material may be superabsorbent polymer (SAP) particles or superabsorbent fibres (SAF).

The superabsorbent material is capable of handling large amounts of wound exudate and retaining the exudate within the upper portion.

In exemplary embodiments, the upper portion may comprise at least one superabsorbent layer and a liquid distribution layer.

The upper portion may have a layered construction. Such a layered pad construction prevents accumulation of body liquids close to the skin and improves the overall liquid handling of the dressing.

The liquid distribution layer may comprise any material having the ability to distribute the exudate in an efficient manner.

For example, the liquid distribution layer may comprise a nonwoven material.

A nonwoven material has the ability to distribute fluid throughout the majority of the material such that wound exudate is evaporated across a larger surface area. The evaporation of wound exudate through the backing layer, and the overall breathability of the dressing is thereby improved.

The liquid distribution layer may be arranged on top of the superabsorbent layer.

A dressing having the construction as explained above is suitable for handling large amounts of exudate and for preventing maceration of the wound site, and of the skin surrounding the wound site.

In exemplary embodiments, the upper portion may comprise a masking layer arranged in contact with the backing layer.

The masking layer is configured to mask wound exudate such that a more visually appealing impression is provided. However, wound exudate is still visibly observable on the lower portion of the wound pad. The masking layer is thus only configured to mask the upper portion.

Typically, the backing layer is substantially transparent.

In exemplary embodiments, the lower portion of the wound pad is white.

Accordingly, the visibility of wound exudate that has reached the lower portion is improved.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1 illustrates a top perspective view of a medical dressing according to an exemplary embodiment of the present disclosure.
Figure 2 illustrates a cross-sectional view of the medical dressing of figure 1.
Figures 3a-d are photographs of a reference dressing (Dressing A) used in the test of Example 1 after 15 hours (fig 3a), 40 hours (fig 3b), 44 hours, (fig 3c), and 70 hours (fig 3d), respectively, of liquid exposure at a constant flow rate, as seen from the top (upper row) and the back of the dressing (lower row).
Figures 4a-d are photographs of a dressing according to an exemplary embodiment of the present disclosure (Dressing B) used in the test of Example 1 after 15 hours (fig 3a), 40 hours (fig 3b), 44 hours, (fig 3c), and 70 hours (fig 3d), respectively, of liquid exposure at a constant flow rate, as seen from the top (upper row) and the back of the dressing (lower row).

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

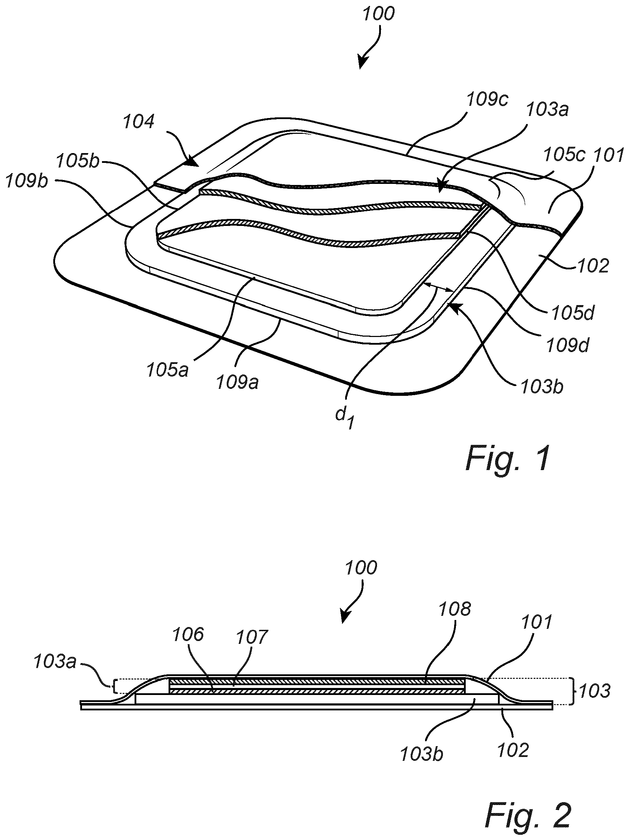

Figure 1 illustrates a wound dressing 100 according to an exemplary embodiment of the present disclosure. The wound dressing 100 comprises a backing layer 101, an adhesive skin contact layer 102 and a wound pad 103 arranged between the backing layer 101 and the adhesive skin contact layer 102, wherein the backing layer 101 and the adhesive skin contact layer 102 are arranged to extend beyond the contour of the wound pad 103 to form a border portion 104, wherein the wound pad 103 comprises an upper portion 103a and a lower portion 103b; the upper portion 103a being arranged in contact with the backing layer 101, wherein the upper portion 103a has a surface area being smaller than the surface area of the lower portion 103b and wherein the upper portion 103a has a higher liquid spreading capacity than the lower portion 103b.

The wound dressing of the present disclosure allows for improvements in guiding caregivers and patients as to when the dressing needs to be changed. Instead of relying on present protocols and change the dressing when the wound exudate has reached at least two or three peripheral wound pad edges, the inventor has shown that the dressing may remain on the skin for an enhanced time period and provided a simple, yet reliable indication means of when the dressing should be replaced.

This is clearly demonstrated in Example 1, where the "standard protocol" of dressing change would have resulted in dressing being discarded too early. As illustrated in Example 1, there is significant capacity left in the wound pad even after three of the peripheral wound pad edges have become wet.

With a dressing of the present disclosure, too frequent dressing changes are avoided. The dressing may thus remain on the skin of a patient for a prolonged period and the capacity of the wound pad is optimized. Furthermore, unnecessary costs associated with discarded dressings is reduced.

The term "liquid spreading capacity" means the spreading or distribution of liquid, e.g. wound exudate, across the surface area of a portion or layer of the wound pad. The liquid spreading capacity generally means the spreading or distribution of liquid in the transverse directions.

The upper portion 103a may be attached to the lower portion 103b by adhesion or lamination.

The surface area of the upper portion 103a corresponds to from 60 to 95%, preferably from 70 to 90% of the surface area of the lower portion 103b.

Preferably, the upper portion 103a is disposed centrally on the lower portion 103b.

However, the invention is by no means limited to this configuration. It is also conceivable that the upper portion 103a may be arranged in a skewed or offset manner with respect to the lower portion 103b. In such embodiments, it is preferred to apply the dressing to the skin of a patient such that the "wetness indicator" area (i.e. the area between the peripheral edge(s) of the lower and upper portions) is arranged in the direction of gravity. For example, if the dressing is to be applied to a knee of a patient, the wound exudate will naturally flow downwards. In this regard, the dressing is thus to be applied such that the wetness indicator area is arranged downwards.

The liquid spreading capacity of the upper portion 103a is at least 50% higher, preferably at least 100% higher, more preferably at least 200% higher than the liquid spreading capacity of the lower portion 103b.

The liquid spreading capacity gradient between the upper 103a and lower 103b portions is preferably as large as possible. This is to provide a reliable means to indicate saturation of the wound pad, and a trustworthy indication to the caregiver(s) with respect to when the dressing needs to be replaced.

In embodiments, the liquid spreading capacity of the upper portion 103a may be at least 300% higher than the liquid spreading capacity of the lower portion 103b.

As illustrated in figure 1, the upper portion is defined by peripheral edges 105a-d. In figure 1, a square shaped dressing is illustrated. However, the dressing is by no means limited to a square shape, but any shape is conceivable. For example, the dressing may be rectangular, oval, circular etc. The dressing may also comprise a plurality of protruding portions, e.g. to fit with the sacrum or heel of a wearer.

The peripheral edges 105a-d of the upper portion 103a (and of the dressing) may thus be straight or curved.

The lower portion 103b may be arranged to extend around the peripheral edges 105a-d of the upper portion 103a.

The lower portion is defined by peripheral edges 109a-d. The area defined between the peripheral edges 105a-d of the upper portion and the peripheral edges 109a-d of the lower portion represents the "wetness indicator area". When wound exudate is visible in this area, the caregiver is guided to discard the dressing and replace it with a new one.

Typically, the upper portion 103a is arranged centrally on the lower portion 103b.

The lower portion 103b may be arranged to extend a distance, d1, around the peripheral edges 105a-d of the upper portion 103a, wherein the distance, d1, may be from 3 to 20 mm, preferably from 4 to 12 mm.

The distance, d1, may vary depending on the size of the dressing. If d1 is too large, the liquid handling capacity (and the wear time) may be impaired. If d1 is too small, the wound exudate may be more difficult to recognize on the lower portion. The distance, d1, may be substantially constant around the peripheral edges 105a-d. Alternatively, the distance, d1, may vary slightly around the peripheral edges 105a-d.

The upper portion 103a may have a higher liquid retention capacity than the lower portion 103b.

As used herein, the term "liquid retention capacity" means the ability of the wound pad to hold and retain wound liquid. The liquid retention capacity may be measured by any standard retention method known to the skilled person. The liquid retention capacity may be measured in the absence or presence of a force, pressure or centrifugation.

For example, the liquid retention capacity of the upper portion 103a may be at least 50% higher, preferably at least 100% higher, more preferably at least 200% higher than the liquid retention capacity of the lower portion.

This construction is beneficial to optimize the overall liquid handling capacity of the dressing and to prevent back-flow of wound exudate from the upper portion 103a to the lower portion 103b.

The lower portion 103b is preferably designed with a rapid liquid acquisition time.

In this regard, the lower portion 103b may comprise a hydrophilic and porous material.

It is also conceivable that the lower portion 103b is provided with incisions or perforations to enhance the liquid acquisition into the upper portion of the wound dressing.

The lower portion 103b may comprise a hydrophilic polyurethane foam.

The thickness, as measured in dry conditions of the hydrophilic polyurethane foam may be in the range of from 1 to 6 mm, e.g. from 1.5 to 4 mm.

A hydrophilic polyurethane foam typically has a short liquid inlet time, a relatively poor liquid spreading capacity, and is capable of quickly acquiring and transporting the wound exudate towards the upper portion 103a of the wound pad.

The polyurethane foam may comprise pores having an average pore size in the range of from 30 and 1000 µm.

The upper portion 103a may comprise a superabsorbent material.

The superabsorbent material may be in the form of particles, fibers, flakes or similar.

The superabsorbent material may comprise superabsorbent polymers (SAP). Superabsorbent polymers are constituted by water-swellable and water insoluble polymers capable of absorbing large quantities of fluid.

As illustrated in figure 1 and figure 2, the upper portion may have a layered construction.

This is to optimize the liquid handling capacity of the wound dressing.

For example, the upper portion may comprise at least one superabsorbent layer 106 and a liquid distribution layer 107.

**In** figure 2, the superabsorbent layer 106 is arranged below the liquid distribution layer 107.

Such a layered pad construction prevents accumulation of body liquids close to the skin and improves the liquid handling of the dressing. Most wounds will contain some exudate, but the level of exudate may vary. **In** a chronic wound, the exudate production may be very large due to an ongoing inflammation. A dressing having the construction as defined above is suitable for handling large amounts of exudate and for preventing maceration of the skin surrounding the wound.

Furthermore, the wound pad construction of the present disclosure is beneficial from a microclimate point of view. Moisture absorbed into the dressing, through the lower portion is quickly transported away from the skin to the superabsorbent layer 106. The liquid distribution layer 107 distributes the wound exudate absorbed by the superabsorbent layer 106 across a large surface area, such that the evaporation of wound exudate through the backing layer is improved. This improves the breathability of the dressing and secures that accumulation of body liquids close to the skin is avoided.

The liquid distribution layer 107 may be a fibrous material.

For example, the liquid distribution layer 107 may comprise a nonwoven.

A nonwoven liquid distribution layer 107 has the ability to distribute fluid throughout the majority of the material. The liquid distribution layer 107 aids in driving the fluid away from the wound site and from the lower portion 103b, while at the same time securing that the maximum capacity of the wound pad is utilized.

The liquid distribution layer 107 may comprise a meltblown, spunbond or a spunlaced nonwoven. Examples of suitable polymers for use in the nonwoven are polyethylene, polyesters, polypropylene and other polyolefin homopolymers and copolymers. For example, nonwoven webs comprising thermoplastic fibers of polypropylene and polyethylene fibres or mixtures thereof may be used. The webs may have a high content of thermoplastic fibres and contain at least 50%, e.g. at least 70% thermoplastic fibres. The nonwoven may be a mixture of polyester and viscose, e.g. in a 70:30 ratio. The basis weight of the nonwoven may be in the range of from 10 to 80 g/m2, e.g. of from 20 to 50 g/m2. The liquid spreading layer may also be a spunbond- meltblown or spunbond-meltblown-spunbond (SMS) web.

The layers of the upper portion 103a of the wound pad may be attached by adhesion or lamination.

It is also conceivable that the upper portion 103a of the wound pad comprises a compressed hydrophilic polyurethane foam. For example, the upper portion 103a may comprise a plurality of compressed depressions (not shown). The plurality of compressed depressions improves the spreading capacity of the polyurethane foam. **In** other words, the compressed depressions improve the ability of the polyurethane foam to spread the liquid in the transverse directions.

As illustrated in figure 2, the upper portion 103b may comprise a masking layer 108 arranged in contact with the backing layer 101.

The provision of a masking layer as a top layer of the upper portion 103a of the wound pad masks wound exudate, which may yield a more visually appealing impression. The provision of a masking layer does not compromise the ability to indicate to a caregiver that the dressing needs to be changed since the exudate will still be observable on the edges of the underlying lower portion 103b.

The masking layer may be colored layer, which masks the blood and any other wound fluid in the wound pad. For example, the masking layer may have a grey color. The masking layer may be a layer contributing to an improved liquid handling of the wound dressing. For example, the masking layer may be a nonwoven layer.

The backing layer 101 is preferably substantially transparent.

As used herein, the term "substantially transparent" means that the wound exudate is visibly observable through the backing layer. **In** other words, wound exudate on the edges of the lower portion is recognizable through the backing layer. The backing layer may be completely transparent, semi-transparent or translucent.

The backing layer may be a film, a laminate or a sheet that is vapor permeable. Examples of suitable materials for the backing layer include, but are not limited to polyurethane, polyethylene or polyamide films, silicone films, polyester based nonwoven materials, and laminates of polyester-based nonwoven materials and polyurethane films. Suitably, the backing layer is a polyurethane film having a thickness of from 5 to 40 µm, e.g. from 15 to 25 µm.

The lower portion 103b of the wound pad may be white or substantially white.

The white color may enhance the visualization of wound exudate present on the edges of the lower portion.

The adhesive skin contact layer 102b typically comprises a silicone-based adhesive.

Preferably, the adhesive skin contact layer 102 comprises a polymeric film provided with an adhesive silicone gel coating; the silicone gel coating being arranged to contact the skin of a patient during use.

An adhesive skin contact layer comprising a silicone gel is skin-friendly and easy to remove without causing trauma. It is sufficiently adherent to skin such that the dressing stays in place, yet is configured to maintain its adherence with repeated removal and re-application.

The polymeric film simplifies the manufacturing process and provides stability and integrity to the adhesive skin contact layer 102.

The polymeric film may be a breathable film and may comprise e.g. polyethylene, polyamide, polyester or polyurethane. Preferably, the polymeric film comprises polyurethane. The thickness of the polyurethane film may be from 15 to 100 µm, e.g. from 20 to 80 µm, preferably from 20 to 60 µm.

Examples of suitable silicone gels for use in the adhesive silicone layer include the two component RTV systems, such as Liveo MG-7-9960 (DuPont), and SilGel 612 (Wacker Chemie AG) mentioned herein, as well as NuSil silicone elastomers. In embodiments of the invention the adhesive may comprise a soft silicone gel having a softness (penetration) of from 8 to 22 mm, e.g. from 12 to 17 mm, as measured by a method based on ASTM D 937 and DIN 51580, the method being described in European Patent Application No 14194054.4. The thickness of the adhesive layer is typically at least 20 µm. The thickness of the adhesive layer may be from 30 to 200 µm.

The adhesive skin contact layer 102 may comprise a plurality of perforations. The perforations typically extend through both the polymeric film and the adhesive silicone gel layer. The perforations allow for a quick absorption into the lower portion of the wound pad without compromising the tight fit to the skin.

The wound dressing of the present disclosure is suitable for use with any wound, incision or scar. The wound dressing may also be useful in negative pressure wound therapy (NPWT). In such cases, the wound dressing may be referred to as an NPWT dressing.

In embodiments where the wound dressing is an NPWT dressing, the dressing may comprise means to connect the dressing to a negative pressure source, and optionally a canister. For example, the backing layer may comprise a coupling member and a tubing assembly configured to connect the dressing to the negative pressure source (and to the canister, if present), wherein the tubing assembly is connected to the coupling member of the wound dressing.

In order to secure transmission of negative pressure, a portion of the wound pad underlying the coupling member may comprise an aperture extending into at least a part of the wound pad, e.g. through the upper portion of the wound pad.

### Example 1:

Comparative tests were carried out utilizing a reference dressing A, and a dressing according to the present disclosure (dressing B).

The dressings used in the test (Dressing A, and Dressing B, respectively) were similar in construction, but differed with respect to the size of the upper portion of the wound pad. Both dressing A and B comprised, from top-to-bottom, a polyurethane backing layer, a wound pad comprising a nonwoven distribution layer, a superabsorbent layer comprising superabsorbent fibers, and a polyurethane foam layer, and an adhesive skin contact layer comprising a polyurethane film and a silicone gel adhesive. The surface area of the upper portion of Dressing B (comprising the nonwoven layer and the superabsorbent layer) was 75% of the surface are of the lower portion of the wound pad (the polyurethane layer).

Comparative performance tests of the dressings were conducted using a simulated wound lab model and simulated wound fluid (SWF). The wound model consists of a transparent polycarbonate plate placed in 60° inclination with a centered fluid inlet hole (10mm in diameter). The dressings were placed center-to-center.

The SWF consisted of horse serum solution (Håtunalab AB, Bro, Sweden), diluted 1:1 with solution A (Sol A recipe is described in EN13726-1). To enhance the color of the fluid a few droplets of red ink was added to the solution. The SWF was pumped into the wound model at 0.5ml/h, by using a syringe pump, 50ml plastic syringes (Becton Dickinson Plastipak with Luer-Lok^{™}) and plastic tube with Luer Lok^{™} connector (B. Braun Original Perfusor^{®} Line Type IV-standard PVC, Melsungen, Germany). The tests were performed in controlled lab environment i.e. 23°C (+/- 2°C and 50% RH (+/-5%).

Photos were taken from both sides (top and bottom) of the dressing at the end of each test to document the liquid distribution in the wound pad.

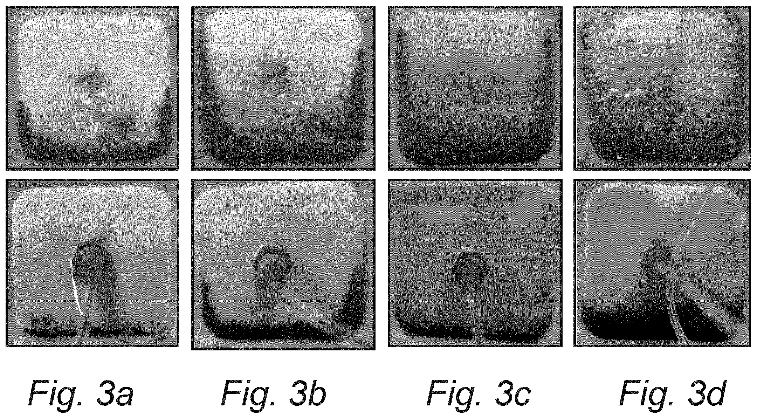

Figures 3a-d illustrate the fluid spreading for dressing A at 15h (figure 3a), 40h, (figure 3b), 44h (figure 3c) and 70h (figure 3d).

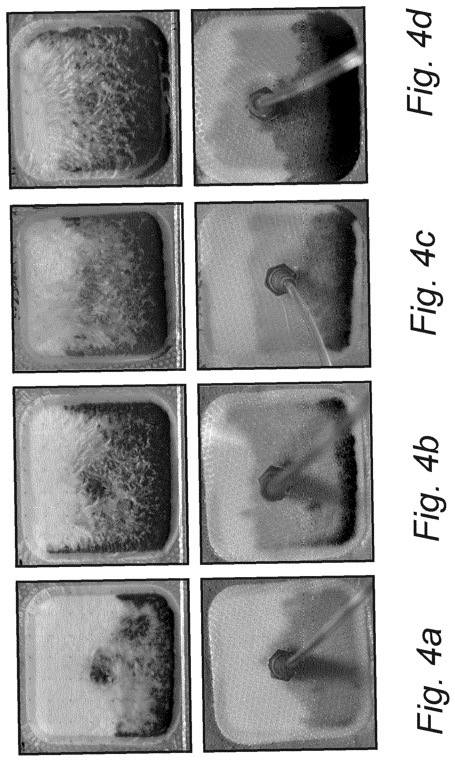

Figures 4a-d illustrate the fluid spreading for dressing B at 15h (figure 4a), 40h, (figure 4b), 44h (figure 4c) and 70h (figure 4d).

To identify absorption problems, and liquid leakage problems, tests were run for different time settings and then evaluated on three different leakage parameters. These leakage parameters were: 1) external leakage outside the border of the dressing 2) pooling, i.e. liquid residuals under dressing at removal from the test equipment, and 3) retention test to identify fluid reflux when the dressings were exposed to an applied external force of 40 mmHg.

The retention test was performed by placing the removed dressing onto a holed steel plate, with a 40% open area. A rigid polycarbonate plate, that covered the full dressing size, was applied onto the dressing, and weights correlated to 40 mmHg were placed onto the plate for 30 seconds. The dressing was weighed before and after the retention test and the difference in weight was documented.

The dressings were evaluated at 15h, 40h, 44h and 70h respectively.
As can be seen in Figure 3a, at least three of the peripheral wound pad edges were wetted already after 15 hours for Dressing A. In applying standard clinical protocols, this would have been an indication of dressing change.

For dressing B, the peripheral edges of the upper portion of the wound pad were also wetted after 15 hours. However, the lower portion of the wound pad remained unwetted until 44 hours, where fluid first reached the peripheral edge of the lower wound pad portion (figure 4c). This is an indication that the dressing needs to be changed.

Furthermore, the leakage indicator tests performed showed no external leakage, pooling or retention leakage (i.e. identified as ≤ 0.02g) at 15 hours, 40 hours and 44 hours. for dressings A and B. This is a confirmation that a dressing according to the present disclosure (dressing B) does not have any leakage problem when first indication of "dressing change" is observed i.e. at 44h. Accordingly, dressing B can stay on the skin up to 3 times longer compared to the recommended standard dressing change protocols.

However, at 70h both dressing A and B showed a clear retention leakage (0.44g, and 0.96 g, respectively), which shows that the methodology can identify leakage problems when a dressing becomes too saturated in this simulated wound lab model.

This way, a simple and reliable means for indicating to a caregiver and/or a patient of when the dressing is to be changed is provided.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A wound dressing (100) comprising a backing layer (101), an adhesive skin contact layer (102) and a wound pad (103) arranged between said backing layer (101) and said adhesive skin contact layer (102), wherein said backing layer (101) and said adhesive skin contact layer (102) are arranged to extend beyond the contour of said wound pad (103) to form a border portion (104), wherein said wound pad (103) comprises an upper portion (103a) and a lower portion (103b); said upper portion (103a) being arranged in contact with said backing layer (101), **characterized in that** said upper portion (103a) has a surface area being smaller than the surface area of said lower portion (103b), wherein the surface area of said upper portion (103a) corresponds to from 60 to 95%, preferably from 70 to 90% of the surface area of said lower portion (103b), and wherein said upper portion (103a) has a higher liquid spreading capacity than said lower portion (103b), wherein the liquid spreading capacity of said upper portion (103a) is at least 50% higher, preferably at least 100% higher, more preferably at least 200% higher than the liquid spreading capacity of said lower portion (103b).

2. The wound dressing (100) according to claim 1, wherein said upper portion (103a) is defined by peripheral edges (105a-d) and wherein said lower portion (103b) is arranged to extend around the peripheral edges (105a-d) of said upper portion (103a).

3. The wound dressing (100) according to claim 2, wherein said lower portion (103b) is arranged to extend a distance, d1, around said peripheral edges (105a-d) of said upper portion (103a), wherein said distance, d1, is from 3 to 20 mm, preferably from 4 to 12 mm.

4. The wound dressing (100) according to any one of the preceding claims, wherein said upper portion (103a) has a higher liquid retention capacity than said lower portion (103b).

5. The wound dressing (100) according to any one of the preceding claims, wherein said upper portion (103a) has a higher absorption capacity than said lower portion (103b).

6. The wound dressing (100) according to any one of the preceding claims, wherein said lower portion (103b) comprises a hydrophilic and porous material.

7. The wound dressing (100) according to any one of the preceding claims, wherein said lower portion (103b) comprises a polyurethane foam.

8. The wound dressing (100) according to any one of the preceding claims, wherein said upper portion (103a) comprises a superabsorbent material.

9. The wound dressing (100) according to any one of the preceding claims, wherein said upper portion (103a) comprises at least one superabsorbent layer (106) and a liquid distribution layer (107).

10. The wound dressing (100) according to claim 9, wherein said liquid distribution layer (107) comprises a nonwoven material.

11. The wound dressing according to any one of the preceding claims, wherein said upper portion (103b) comprises a masking layer (108) arranged in contact with said backing layer (101).

12. The wound dressing (100) according to any one of the preceding claims, wherein said backing layer (101) is substantially transparent.

13. The wound dressing according to any one of the preceding claims, wherein said lower portion (103b) of said wound pad (103) is white.

## Patentansprüche

1. Wundverband (100), der eine Trägerschicht (101), eine haftende Hautkontaktschicht (102) und ein Wundkissen (103) umfasst, das zwischen der Trägerschicht (101) und der haftenden Hautkontaktschicht (102) angeordnet ist, wobei die Trägerschicht (101) und die haftende Hautkontaktschicht (102) so angeordnet sind, dass sie sich über den Umriss des Wundkissens (103) hinaus erstrecken und somit einen Grenzabschnitt (104) bilden, wobei das Wundkissen (103) einen oberen Abschnitt (103a) und einen unteren Abschnitt (103b) umfasst; wobei der obere Abschnitt (103a) im Kontakt mit der Trägerschicht (101) angeordnet ist, **dadurch gekennzeichnet, dass** der obere Abschnitt (103a) einen Flächeninhalt aufweist, der kleiner ist als der Flächeninhalt des unteren Abschnitts (103b), wobei der Flächeninhalt des oberen Abschnitts (103a) von 60 bis 95%, vorzugsweise von 70 bis 90% des Flächeninhalts des unteren Abschnitts (103b) entspricht, und wobei der obere Abschnitt (103a) eine größere Flüssigkeitsverteilleistung aufweist als der untere Abschnitt (103b), wobei die Flüssigkeitsverteilleistung des oberen Abschnitts (103a) mindestens 50% höher, vorzugsweise mindestens 100% höher, bevorzugter mindestens 200% höher ist als die Flüssigkeitsverteilleistung des unteren Abschnitts (103b).

2. Wundverband (100) nach Anspruch 1, wobei der obere Abschnitt (103a) durch umlaufende Ränder (105a-d) definiert ist und wobei der untere Abschnitt (103b) so angeordnet ist, dass er um die umlaufenden Ränder (105a-d) des oberen Abschnitts (103a) verläuft.

3. Wundverband (100) nach Anspruch 2, wobei der untere Abschnitt (103b) so angeordnet ist, dass er in einem Abstand d1 um die umlaufenden Ränder (105a-d) des oberen Abschnitts (103a) herum verläuft, wobei der Abstand d1 von 3 bis 20 mm, vorzugsweise von 4 bis 12 mm beträgt.

4. Wundverband (100) nach einem der vorhergehenden Ansprüche, wobei der obere Abschnitt (103a) ein höheres Flüssigkeitsrückhaltevermögen aufweist als der untere Abschnitt (103b).

5. Wundverband (100) nach einem der vorhergehenden Ansprüche, wobei der obere Abschnitt (103a) eine höhere Aufnahmekapazität aufweist als der untere Abschnitt (103b).

6. Wundverband (100) nach einem der vorhergehenden Ansprüche, wobei der untere Abschnitt (103b) ein hydrophiles und poröses Material umfasst.

7. Wundverband (100) nach einem der vorhergehenden Ansprüche, wobei der untere Abschnitt (103b) einen Polyurethanschaum umfasst.

8. Wundverband (100) nach einem der vorhergehenden Ansprüche, wobei der obere Abschnitt (103a) ein superabsorbierendes Material umfasst.

9. Wundverband (100) nach einem der vorhergehenden Ansprüche, wobei der obere Abschnitt (103a) mindestens eine superabsorbierende Schicht (106) und eine Flüssigkeitsverteilschicht (107) aufweist.

10. Wundverband (100) nach Anspruch 9, wobei die Flüssigkeitsverteilschicht (107) ein Vlies umfasst.

11. Wundverband nach einem der vorhergehenden Ansprüche, wobei der obere Abschnitt (103b) eine Abdeckschicht (108) im Kontakt mit der Trägerschicht (101) aufweist.

12. Wundverband (100) nach einem der vorhergehenden Ansprüche, wobei die Trägerschicht (101) im Wesentlichen transparent ist.

13. Wundverband nach einem der vorhergehenden Ansprüche, wobei der untere Abschnitt (103b) des Wundkissens (103) weiß ist.

## Revendications

1. Pansement pour plaies (100) comprenant une couche support (101), une couche adhésive de contact avec la peau (102) et un tampon (103) placé entre ladite couche support (101) et ladite couche adhésive de contact avec la peau (102), dans lequel ladite couche support (101) et ladite couche adhésive de contact avec la peau (102) sont agencées pour s'étendre sur le contour dudit tampon (103) pour former une partie de bordure (104), ledit tampon (103) comprenant une partie supérieure (103a) et une partie inférieure (103b) ; ladite partie supérieure (103a) étant disposée en contact avec ladite couche support (101), **caractérisé en ce que** ladite partie supérieure (103a) a une aire de surface qui est inférieure à l'aire de surface de ladite partie inférieure (103b), l'aire de surface de ladite partie supérieure (103a) correspondant à 60 à 95 %, de préférence à 70 à 90 %, de l'aire de surface de ladite partie inférieure (103b), et ladite partie supérieure (103a) ayant une capacité d'étalement de liquide supérieure à celle de ladite partie inférieure (103b), la capacité d'étalement de liquide de ladite partie supérieure (103a) étant supérieure d'au moins 50 %, de préférence d'au moins 100 %, mieux encore d'au moins 200 %, à la capacité d'étalement de liquide de ladite partie inférieure (103b).

2. Pansement pour plaies (100) selon la revendication 1, dans lequel ladite partie supérieure (103a) est définie par des bords périphériques (105a-d) et ladite partie inférieure (103b) est disposée de manière à s'étendre autour des bords périphériques (105a-d) de ladite partie supérieure (103a).

3. Pansement pour plaies (100) selon la revendication 2, dans lequel ladite partie inférieure (103b) est disposée de manière à s'étendre sur une distance dl autour desdits bords périphériques (105a-d) de ladite partie supérieure (103a), lequel ladite distance dl étant de 3 à 20 mm, de préférence de 4 à 12 mm.

4. Pansement pour plaies (100) selon l'une quelconque des revendications précédentes, dans lequel ladite partie supérieure (103a) a une capacité de rétention de liquide supérieure à celle de ladite partie inférieure (103b).

5. Pansement pour plaies (100) selon l'une quelconque des revendications précédentes, dans lequel ladite partie supérieure (103a) a une capacité d'absorption supérieure à celle de ladite partie inférieure (103b).

6. Pansement pour plaies (100) selon l'une quelconque des revendications précédentes, dans lequel ladite partie inférieure (103b) comprend un matériau hydrophile et poreux.

7. Pansement pour plaies (100) selon l'une quelconque des revendications précédentes, dans lequel ladite partie inférieure (103b) comprend une mousse de polyuréthane.

8. Pansement pour plaies (100) selon l'une quelconque des revendications précédentes, dans lequel ladite partie supérieure (103a) comprend un matériau super-absorbant.

9. Pansement pour plaies (100) selon l'une quelconque des revendications précédentes, dans lequel ladite partie supérieure (103a) comprend au moins une couche super-absorbante (106) et une couche de répartition de liquide (107).

10. Pansement pour plaies (100) selon la revendication 9, dans lequel ladite couche de répartition de liquide (107) comprend un matériau non tissé.

11. Pansement pour plaies selon l'une quelconque des revendications précédentes, dans lequel ladite partie supérieure (103b) comprend une couche de masquage (108) disposée en contact avec ladite couche support (101).

12. Pansement pour plaies (100) selon l'une quelconque des revendications précédentes, dans lequel ladite couche support (101) est sensiblement transparente.

13. Pansement pour plaies selon l'une quelconque des revendications précédentes, dans lequel ladite partie inférieure (103b) dudit tampon (103) est blanche.
